(19) Europäisches Patentamt
European Patent Office
Office européen des brevets

(11) Veröffentlichungsnummer: **0 041 612**
**B1**

(12) **EUROPÄISCHE PATENTSCHRIFT**

(45) Veröffentlichungstag der Patentschrift: **15.02.84**

(51) Int. Cl.³: **C 07 J 7/00**, C 07 J 41/00

(21) Anmeldenummer: **81103333.1**

(22) Anmeldetag: **02.05.81**

(54) **Verfahren zur Herstellung von 20-Carbonyl-Steroiden durch Abbau von Steroid-C22-Carbonsäuren.**

(30) Priorität: **09.06.80 AT 3031/80**

(43) Veröffentlichungstag der Anmeldung: **16.12.81 Patentblatt 81/50**

(45) Bekanntmachung des Hinweises auf die Patenterteilung: **15.02.84 Patentblatt 84/7**

(84) Benannte Vertragsstaaten: **FR NL**

(56) Entgegenhaltungen:
**US - A - 2 492 193**

**HOUBEN-WEYL: "Methoden der Organischen Chemie, IV. Auflage, Band VII/2b, Ketone, Teil II, 1976, Seiten 1364-1365, Georg Thieme Verlag Stuttgart, DE**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Preuss, Wolfgang, Dr., Finkenweg 12, D-4019 Monheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 041 612 B1

# Verfahren zur Herstellung von 20-Carbonyl-Steroiden durch Abbau von Steroid-C22-carbonsäuren

Im Bereich der pharmakologisch bedeutungsvollen Steroidverbindungen und bei der Synthese solcher Verbindungen spielen Steroide eine besondere Rolle, die in 17-Stellung als Substituenten den $CH_3CO$-Rest des Progesterons aufweisen. Dieser Acetylrest in Verbindungen vom Progesterontyp kann durch Aufbau — ausgehend von 17-on-Steroiden — gewonnen werden. Zunehmend bekommen heute jedoch Syntheseverfahren Bedeutung, in denen — insbesondere ausgehend von natürlichen Steroidverbindungen pflanzlichen oder tierischen Ursprungs — der ursprünglich längerkettige Substituent in 17-Stellung einem partiellen Abbau zugeführt wird. So beschreibten beispielsweise die offengelegten europäischen Patentanmeldungen Nrn. 004913 und 0015308 ein Verfahren zur Herstellung von 17 C-Steroid-α-propionsäureverbindungen — insbesondere zur Herstellung von 3-Oxo-pregna-4-en-20-carbonsäure (Δ 1,4-BNC) und/oder 3-Oxo-pregna-1,4-dien-20-carbonsäure (Δ 1,4-BNC) — durch mikrobiellen Seitenkettenabbau an 17 C-Seitenketten-Steroidsubstraten natürlichen Ursprungs.

BNC-Verbindungen dieser und verwandter Art sind noch um das Kohlenstoffatom in 22-Stellung reicher — das hier als Carboxylgruppe vorliegt — als Steroidverbindungen vom Progesterontyp. Zur Verwertung entsprechender BNC-Verbindungen für die weitere Synthese von pharmakologisch interessanten Steroidverbindungen bedarf es des Abbaues der Carboxylgruppe in C22 beziehungsweise der Umwandlung dieses am C20 vorliegenden Substituenten in die C20-Carbonylgruppe.

Bekannt ist für die 3-Acetoxy-5-en-BNC, dass die als Substituent in C20 vorliegende Carboxylgruppe durch einen klassischen Säureabbau nach Curtius entfernt werden kann, siehe «Ber.», 88 (1955) 883. Als Produkt dieses Abbaues bildet sich die 20-Amino-Steroidverbindung. Ihre Umwandlung in die 20-Ketoverbindung des Progesterontyps verlangt einen nicht unbeträchtlichen Aufwand.

Im Rahmen des Abbaues von C17-Seitenketten-Steroidsubstraten, insbesondere natürlichen Ursprungs, durch Mikroorganismen sind heute eine Reihe wichtiger BNC-Verbindungen zugänglich geworden, die eine zusätzliche en-Bindung in 17(20)-Stellung aufweisen. So schildert die US-PS Nr. 3994933 die Herstellung von 3-Oxo-pregna-4,17(20)-dien-20-carbonsäure (Δ 4,17-BNC), ihren niederen Alkylestern und pharmakologisch verträglichen Salzen. Die Säure wird durch mikrobiellen Seitenkettenabbau von 17 C-Steroidverbindungen gewonnen.

In den europäischen Patentanmeldungen Nrn. 81.100146.0/33439 und 81.100145.2/34248 der Anmelderin wird die Gewinnung von 20-Carboxy-pregna-1,4,17(20)-trien-3-on (Δ 1,4,17-BNC) durch mikrobiellen Seitenkettenabbau an 17 C-Seitenketten-Steroidsubstraten beschrieben. Geschildert ist dort weiterhin die Umwandlung der 20-Carbonsäuregruppe in den entsprechenden Ester beziehungsweise das Carbonsäurechlorid.

Die AT-PS Nr. 369032, «Neue Δ 17(20)-BNC-Verbindungen und Verfahren zu ihrer Herstellung», Priorität Österreich, A 2534/80 vom 12.5.1980 sowie AT-PS Nr. 368759, «Neue Pregnan-20-Carbonsäurederivate und Verfahren zu ihrer Herstellung», Priorität Österreich A 2535/80 vom 12.5.1980 schildern neue Δ 4,17(20)- und Δ 4,17(20)-BNC-Verbindungen der allgemeinen Formel

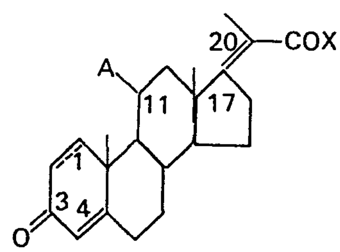

in der bedeuten: A = α-Hydroxyl, β-Hydroxyl oder gemeinsam mit dem durch A substituierten C-Atom eine Carbonylgruppe und X = Hydroxyl, OK, wobei K eine salzbildende Gruppe ist, OR (R = ein Kohlenwasserstoffrest mit vorzugsweise nicht mehr als 20 C-Atomen und insbesondere ein niederer Alkylrest) Halogen, insbesondere Chlor oder Brom, oder $NH_2$.

In diesen Anmeldungen wird auch das Verfahren zur Einführung der Sauerstoff-Funktion in 11-Stellung geschildert.

Solche in 11-Stellung mit einer Sauerstoff-Funktion versehenen BNC-Verbindungen — insbesondere die 11 β-hydroxylierten Derivate — können Ausgangspunkt für eine anschliessende Dehydratisierung unter Einführung einer weiteren Doppelbindung in das Steroidringgerüst sein. Diese Doppelbindung bildet sich unter geeigneten Bedingungen bevorzugt in 9(11)-Stellung aus, siehe hierzu beispielsweise die europäische Patentanmeldung Nr. 81.103332.3/40347, «Neue Steroid-20-Carbonsäureverbindungen und Verfahren zu ihrer Herstellung», Priorität Österreich A 2629/80 vom 16.5.1980.

Die vorliegende Erfindung geht von der Aufgabe aus, ein Verfahren zur Verfügung zu stellen, durch das in vereinfachter Weise die Umwandlung der als Substituent in 20-Stellung vorliegenden Carboxylgruppe in C22 zur 20-Carbonylgruppierung gelingt. Dieses Verfahren soll insbesondere den unmittelbaren Übergang von der C22-Steroidcarbonsäure zur C21-Steroidverbindung vom Progesterontyp ermöglichen, ohne dass es der intermediären Isolierung von Reaktionszwischenprodukten bedürfte.

Die Lehre der Erfindung geht von der Erkenntnis aus, dass insbesondere Steroidverbindungen mit der Δ 17(20)-Struktur zur Lösung der erfindungsgemässen Aufgabe geeignet sind.

Gegenstand der Erfindung ist dementsprechend ein vereinfachtes Verfahren zum Abbau von Steroid-C22-carbonsäuren zu C20-Carbonyl-Steroiden, wobei dieses Verfahren dadurch gekennzeichnet ist, dass man Δ 17(20)-Steroid-22-carbonsäuren dem Curtius-Abbau oder dem Carboxy-Inversionsabbau unterwirft. Vorzugsweise wird durch dieses Verfahren ohne Isolierung von Reaktionszwischenprodukten unmittelbar die um das Kohlenstoffatom in 22-Stellung ärmere entsprechende Steroidverbindung vom Progesterontyp gewonnen.

Als Ausgangsmaterial für das erfindungsgemässe Verfahren sind Steroidverbindungen geeignet, die in 17-Stellung als Substituenten den Rest der Propionsäure enthalten, wobei dieser Rest in α-Stellung mit einer olefinischen Doppelbindung an C17 des Steroidrings gebunden ist. Wie noch im einzelnen geschildert wird, führt bei Ausgangsverbindungen dieser Art sowohl der Curtius-Abbau wie der Carboxy-Inversions-Abbau unmittelbar zur Progesteronstruktur in C17.

Als Ausgangsmaterial für das erfindungsgemässe Verfahren sind insbesondere Δ 4(5),17(20)-BNC-Verbindungen geeignet, die gegebenenfalls auch weitere olefinische Doppelbindungen enthalten können. Besonders geeignete Verbindungen der zuletzt genannten Art enthalten zusätzliche en-Bindungen in 1(2)-Stellung und/oder in 9(11)-Stellung. Besonders wichtige Ausgangsmaterialien für die Durchführung der Lehre der Erfindung sind damit die folgenden BNC-Verbindungen: Δ 4,17(20)-BNC, Δ 1,4,17(20)-BNC, Δ 4,9(11),17(20)-BNC und Δ 1,4,9(11),17(20)-BNC. Diese Ausgangsverbindungen sind über die eingangs genannten Veröffentlichungen und Anmeldungen der Anmelderin zugänglich.

Für die Durchführung der erfindungsgemässen Lehre sind insbesondere aber auch die in 11-Stellung mit Sauerstoff funktionalisierten Derivate der zuvor genannten Verbindungen geeignet, soweit sie existent sind. In 11-Stellung kann dabei eine Hydroxylgruppe vorliegen — in diesem Fall kann sowohl die 11 α-Hydroxylgruppe als insbesondere die 11 β-Hydroxylgruppe vorliegen —, es ist aber auch möglich, dass in 11-Stellung die Ketogruppe vorliegt. Wichtig sind insbesondere die entsprechend mit Sauerstoff funktionalisierten BNC-Verbindungen der Δ 4,17(20)-BNC und der Δ 1,4,17(20)-BNC. Ausgangsverbindungen der hier betroffenen Art sind ebenfalls gemäss der Lehre der bereits genannten Patentanmeldungen zugänglich.

Der Carboxylgruppenabbau in C22 führt erfindungsgemäss sowohl auf dem Weg des Curtius-Abbaus als auf dem Weg des Carboxy-Inversions-Abbaus unmittelbar zur 20-Carbonylgruppierung, wobei sich in der Regel die Isolierung von intermediär auftretenden Zwischenstufen erübrigt. Allenfalls die Isolierung des Säurehalogenids, das auf beiden Verfahrenswegen als Zwischenstufe auftritt, kann von technischer Bedeutung sein, beispielsweise zur Gewinnung reinerer Produkte.

Für die Umwandlung der freien Carbonsäuren in ihre Halogenide gilt das folgende:

Die Überführung von Carbonsäuren in Säurehalogenide — insbesondere Säurechloride — unter Verwendung von Halogenierungsmitteln wie Phosphorhalogenide, Oxalylhalogenid oder insbesondere Thionylhalogenid ist eine an sich seit langem bekannte und allgemein benutzte Reaktion, auch in der Reihe der 20-Carboxy-Pregnan-Derivate.

Wendet man aber die in der Literatur — siehe hierzu beispielsweise «FIAT Final Report» Nr. 996, Seiten 24 ff. sowie P. L. Julian, E. W. Meyer und H. C. Printy, «J. Am. Chem. Soc.», *70*, 887 (1948) — für die Umsetzung von 3-Acetoxy-bis-norcholensäure mit Thionylchlorid angegebenen Reaktionsbedingungen auf beispielsweise Δ 1,4-BNC an, verestert danach das so erhaltene Säurechlorid mit Methanol und analysiert das Rohprodukt, so findet man im Gaschromatogramm einen zusätzlichen Peak und bei der Elementaranalyse einen deutlichen, zunächst nicht erwarteten Cl-Gehalt.

Ähnliches gilt sogar in noch stärkerem Masse für die Verwendung von Oxalylchlorid anstelle von Thionylchlorid.

Der Grund für das Auftreten dieser unerwünschten Verunreinigungen, die die Ausbeute an gewünschtem Produkt deutlich senken und zu Reinigungsproblemen bei weiteren Umsetzungen mit dem Säurechlorid führen können, ist wahrscheinlich eine Chlorierung mit evtl. anschliessender Aromatisierung des A-Ringes im Steroidgerüst, wie sie z.B. für die Umsetzung von Adrosta-1,4-dien-3,17-dion (ADD) mit Oxalchlorid bekannt ist; siehe hierzu G. W. Moersch *et al.*, «J. Org. Chemistry», *29*, 2495 (1964).

Überraschenderweise gelingt die Bildung der erfindungsgemäss gewünschten Säurehalogenide bereits unter aussergewöhnlich milden Reaktionsbedingungen, bei denen die unerwünschte Mitreaktion anderer reaktiver Stellen der zugrunde liegenden ein- oder mehrfach ungesättigten BNC-Struktur noch nicht stattfindet. So zeigte sich das überraschende Ergebnis, das beispielsweise eine praktisch quantitative Säurechloridbildung stattfindet, wenn die folgenden Reaktionsbedingungen eingehalten werden: Reaktionstemperaturen unter 15° C, vorzugsweise unter 5° C, insbesondere im Bereich von 0-5° C, stöchiometrische Mengen der Reaktanten oder nur sehr begrenzter Überschuss des Halogenierungsmittels, der vorzugsweise nicht über 20 Molprozent und insbesondere nicht über 10 Molprozent beträgt, sowie Arbeiten in Gegenwart eines inerten Verdünnungsmittels, gewünschtenfalls in Anwesenheit geringer Mengen eines basischen Katalysators.

Als inerte Lösungsmittel kommen beispielsweise halogenierte Kohlenwasserstoffe oder mit Einschränkungen Ether in Betracht. Geeignete inerte Lösungsmittel sind beispielsweise Methylenchlorid oder Chloroform. Als Halogenierungsmittel kommen Phosphorhalogenide, insbesondere $PCl_3$ oder $PCl_5$ bzw. die entsprechenden Bromide, vor allen Dingen jedoch das Thionylhalogenid, insbesondere Thionylchlorid, in Betracht. Katalytische Mengen einer Base, insbesondere Pyridin oder Dimethylformamid, beschleunigen z.B. bei der Δ 4,17(20)-BNC die Reaktion, sind aber häufig nicht erforderlich. In Einzelfällen kann die Mitverwendung eines Katalysators wünschenswert sein.

Je nach Wahl der sonstigen Verfahrensparameter bzw. der miteinander umzusetzenden Verbindungen können einzelne Verfahrensparameter auch ausserhalb der bisher angegebenen Werte liegen. So kann beispielsweise die Verfahrenstemperatur im Bereich von etwa −20°C bis etwa 75°C liegen, vorausgesetzt, dass bei den höheren Temperaturen des genannten Bereiches durch geeignete sonstige Verfahrensführung das Entstehen unerwünschter Ringhalogenierungsprodukte vermieden wird. Die Menge des Halogenierungsmittels kann — wiederum geeignete Abstimmung der sonstigen Verfahrensbedingungen vorausgesetzt — auch in nicht unbeträchtlichem Überschuss über die stöchiometrisch benötigte Menge hinaus vorliegen. So sind beispielsweise Mengen bis zu 5 Äquivalenten, vorzugsweise bis zu 3 Äquivalenten des Halogenierungsmittels in Sonderfällen brauchbar. Die Reaktion wird üblicherweise bei Normaldruck durchgeführt. Das Halogenierungsmittel wird zweckmässigerweise zu der Lösung der umzusetzenden Steroidverbindung im inerten Lösungsmittel gegeben. Es hat sich als vorteilhaft erwiesen, das Halogenierungsmittel in möglichst reiner Form einzusetzen. Offenbar fördern üblicherweise im Halogenierungsmittel vorliegende Verunreinigungen unerwünschte Nebenreaktionen. Zweckmässig ist beispielsweise eine Reinigung des Halogenierungsmittels mit einer ungesättigten Verbindung wie Leinöl oder besonders Squalen. Diese ungesättigten Komponenten reagieren mit den Verunreinigungen im Halogenierungsmittel und senken damit die Bildung unerwünschter Nebenprodukte auf ein Minimum.

Das Säurehalogenid kann im allgemeinen mit oder ohne zwischengeschalteter Reinigung nach einem der im folgenden ausführlich beschriebenen Wege weiter verarbeitet werden.

Zum Abbau der Carboxylgruppe in 22-Stellung gelten einerseits die umfangreichen Angaben der Literatur im Zusammenhang mit dem Curtius-Abbau von Carbonsäuren über deren Azide zu den nächst niederen primären Aminen.

Die Carbonsäureazide bilden sich im allgemeinen in glatter Reaktion, siehe hierzu beispielsweise Houben Weyl, Methoden der organischen Chemie, (1957), Bd. XI/1, Seiten 862 ff., insbesondere 864.

Im Rahmen der Erfindung hat sich insbesondere die modifizierte Variante des Curtius-Abbaus unter Anwendung der Zweiphasentechnik bewährt, wie sie — für den Abbau von Fettsäuren und dimerisierten Fettsäuren — in der DE-OS Nr. 2245611 beschrieben ist. Diese Variante arbeitet mit einer Zweiphasentechnik der Art, dass das Säurehalogenid in einem mit Wasser im wesentlichen nicht mischbaren organischen Lösungsmittel gelöst wird und die Umsetzung des Acylhalogenids und des Metallazids als Zweiphasenreaktion mit einer wässerigen Lösung des Azids in Gegenwart eines quartären Ammoniumsalzes als Phasentransferkatalysator durchgeführt wird.

Diese Stufe des erfindungsgemässen Verfahrens wird vorzugsweise unter Bedingungen durchgeführt, die einen wesentlichen Abbau des gebildeten Acylazids zum Isocyanat ausschliessen. Vorzugsweise wird bei Temperaturen unter etwa 25°C und insbesondere bei Temperaturen unter etwa 15°C, zweckmässig im Bereich von etwa 0-15°C gearbeitet. Die Reaktionsteilnehmer werden während der Umsetzung vorzugsweise bewegt, z.B. gerührt. Diese Vermischung soll jedoch nicht zur Bildung einer beständigen Emulsion führen. Nach Abschluss der Reaktion kann die das Acylazid enthaltende organische Lösung von der wässerigen Phase abgetrennt werden. Die organische Phase wird nachgewaschen.

Als mit Wasser nicht mischbare organische Lösungsmittel eignen sich beispielsweise aliphatische, alicyclische oder aromatische Kohlenwasserstoffe oder insbesondere halogenierte Kohlenwasserstoffe, wie Methylenchlorid oder Chlorbenzol. Die Konzentration des Acylhalogenids im Lösungsmittel ist nicht kritisch und kann beispielsweise etwa 5 bis 50 Gewichtsprozent betragen. Als Metallazide werden vorzugsweise Alkalimetall- oder Erdalkalimetallazide, insbesondere Kaliumazid und vor allem Natriumazid eingesetzt. Das quartäre Ammoniumsalz enthält am Stickstoff vier beliebige organische Reste, beispielsweise Alkyl- oder Arylreste. Vorzugsweise beträgt die gesamte Kohlenstoffzahl nicht mehr als etwa 30, wobei entsprechende Quartärsalze mit bis zu etwa 20 Kohlenstoffatomen in den das Stickstoffatom alkylierenden Resten besonders geeignet sein können. Kurzkettige Alkylreste mit 1-5 Kohlenstoffatomen können besonders bevorzugt sein. Als Anion können beliebige Reste vorliegen. Halogenidionen können besonders zweckmässig sein. Das quartäre Ammoniumsalz wird üblicherweise in nur geringen Mengen, z.B. Mengen von 0,01-10 Äquivalentprozent, bezogen auf das Säurehalogenid, eingesetzt. Die bevorzugten Verfahrenstemperaturen liegen im Bereich unterhalb etwa 10°C und insbesondere im Bereich von 0-5°C.

Nach Abschluss der Reaktion, Abtrennung der organischen Phase und ihrem Waschen zur Senkung des Gehalts an quartären Ammoniumverbindungen, kann das gebildete Steroid-20-carbonsäureazid bei vorsichtigem Arbeiten durch Entfernung des Lösungsmittels isoliert werden. Üblicherweise wird jedoch die Lösung des Säureazids nach einem der im folgenden geschilderten Wege unmittelbar weiter verarbeitet.

In besonders einfacher Reaktion kann das Carbonsäureazid unmittelbar in die C20-Carbonylverbindung umgewandelt werden. Bekannt ist an sich, dass sich Carbonsäureazide durch Erhitzen mit wässerigen Säuren, insbesondere wässeriger Essigsäure, unmittelbar zu den um ein Kohlenstoffatom ärmeren Aminen abbauen lassen. Im Rahmen der Erfindung fallen diese Amine jedoch nicht an, stattdessen entstehen unmittelbar die gesuchten 20-Carbonylverbindungen. Offenbar lagert sich das intermediär entstehende 17(20)-ungesättigte Amin unmittelbar zum 17(20)-gesättigten 20-Imin um, das unter den Reaktionsbedingungen zur 20-Ketogruppe hydrolysiert. Im ein-

zelnen kann beispielsweise folgendermassen vorgegangen werden:

Die Lösung des gebildeten Säureazids im mit Wasser nicht mischbaren Lösungsmittel wird zu einem Überschuss an wässeriger Essigsäure (Konzentration beispielsweise 50 bis 80 Gew.-%) getropft. Gleichzeitig wird aus der Reaktionsmischung das Lösungsmittel vorsichtig abdestilliert. Es kann unter Umständen zweckmässig sein, das zusammen mit dem Lösungsmittel entfernte Wasser zu ersetzen. Schliesslich steigert man die Reaktionstemperatur auf 60 bis 70°C. Hat die Gasentwicklung nachgelassen, unterwirft man die Mischung einer Wasserdampfdestillation. Der Rückstand wird im Vakuum eingeengt. Mit Wasser nicht mischbares Lösungsmittel und gewünschtenfalls wässerige Alkalilauge werden zugesetzt und das Gemisch bewegt. Anschliessend erfolgt eine Phasentrennung, die organische Phase wird mit Wasser gewaschen und schliesslich zur Trockne eingeengt. Die anfallende rohe 20-Carbonylverbindung kann in konventioneller Weise gereinigt werden.

Gewünschtenfalls kann auch mehrstufig derart gearbeitet werden, dass zunächst eine Umwandlung des Säureazids durch Stickstoffabspaltung zum Isocyanat erfolgt, das dann an sich bekannten Folgereaktionen, z.B. der Umwandlung zur Carbonylverbindung unterworfen werden kann. Es gelten dann die allgemeinen Gesetzmässigkeiten des Curtius-Abbaues (siehe Houben Weyl a.a.O., insbesondere Seiten 862 und 865/866). Gegenüber dem normalen Ablauf des Curtius-Abbaues unterscheidet sich jedoch das erfindungsgemässe Verfahren stets durch die Bildung der 20-Carbonylgruppe statt einer 20-Aminogruppierung.

Überraschenderweise hat sich gezeigt, dass die sterische Konfiguration des so entstehenden Substituenten in C17 der pharmakologisch gewünschten Konfiguration entspricht. Auch dieser Sachverhalt war nicht vorhersehbar.

Die andere Möglichkeit des erfindungsgemässen Verfahrens sieht den Carboxylgruppenabbau mittels der Carboxy-Inversions-Reaktion vor. Hierbei erfolgt die Umsetzung des Säurehalogenids mit einer Persäure und anschliessender Verseifung des aus dem gemischten Anhydrid gebildeten Umlagerungsproduktes. Diese Carboxy-Inversions-Reaktion ist im einzelnen beschrieben in «J. Org. Chemistry», 30, 3760 (1965), D. B. Denney, N. Sherman, «Degradation of Acids to Alcohols by the Carboxy-Inversion Reaction». Überraschenderweise lässt sich dieses Abbauverfahren erfolgreich auch auf Steroidcarbonsäuren bzw. ihre entsprechenden Halogenide anwenden, obwohl das Steroidgerüst dieser Carbonsäuren funktionelle Gruppierungen an mehrfacher Stelle enthält. Diese glatte Anwendbarkeit des an sich bekannten Verfahrenstyps war nicht zu erwarten. Bei den erfindungsgemäss eingesetzten 17(20)-Steroidverbindungen entstehen gleichzeitig wiederum als direkte Verfahrensprodukte die 20-Carbonylderivate vom Progesterontyp.

Auch hier gilt, dass die entstehende Konfiguration in 17-Stellung der pharmakologisch geforderten Konfiguration entspricht. Auch das war nicht vorhersehbar.

Als Persäure zur Bildung des gemischten Säureanhydrids wird erfindungsgemäss bevorzugt m-Chlorperbenzoesäure eingesetzt. Diese Verbindung steht in technischen Mengen zur Verfügung, ist relativ ungefährlich in der Handhabung und bringt einen glatten Reaktionsablauf. Anstelle von m-Chlorperbenzoesäure können aber auch andere Persäuren eingesetzt werden. Bevorzugt sind dabei aromatische Persäuren, insbesondere solche mit einem elektronenziehenden Rest am aromatischen Kern wie p-Nitroperbenzoesäure.

Die Umsetzung des Steroidcarbonsäurechlorids mit der Persäure, insbesondere mit m-Chlorperbenzoesäure wird bei Temperaturen −50°C und Raumtemperatur durchgeführt. Die bevorzugten Reaktionstemperaturen liegen zwischen −10 und −30°C.

Die Umsetzung erfolgt zweckmässig in einem inerten Lösungsmittel in Gegenwart einer Base. Als Lösungsmittel werden beispielsweise halogenierte Kohlenwasserstoffe wie Methylenchlorid oder Chloroform, Ether wie Diethylether oder Tetrahydrofuran, Nitrile wie Acetonitril, Säureamide wie Dimethylformamid oder Ketone wie z.B. Aceton eingesetzt. Die bevorzugten inerten Verdünnungsmittel sind Lösungsmittel mit einer gewissen Polarität. Als Basen sind tertiäre Stickstoffbasen besonders geeignet. Beispiele hierfür sind Pyridin, Methylpyridine, N,N-Dimethylaminopyridin, Triethylamin oder Ethyldiisopropylamin. Es kommen also sowohl ringförmige als auch offenkettige tertiäre Amine in Betracht. Aber auch anorganische basische Komponenten, insbesondere basische Salze aus starken Basen und schwachen, insbesondere flüchtigen Säuren können Verwendung finden. Ein geeignetes Beispiel ist etwa Lithiumcarbonat. Die Base wird vorzugsweise in solchen Mengen eingesetzt, dass eine Bindung des bei der Umsetzung entstehenden Halogenwasserstoffs möglich ist.

Die Reaktionsführung erfolgt vorzugsweise derart, dass der Lösung des Steroidcarbonsäurehalogenids im inerten Lösungsmittel nach Kühlung auf die gewünschte Anfangstemperatur der Reaktion — z.B. nach Kühlung auf −30°C — die Persäure und dann anschliessend unter Rühren und Kühlen vorsichtig portionsweise die basische Komponente zugesetzt werden. Man lässt dann über einen längeren Zeitraum, z.B. über Nacht auf Raumtemperatur erwärmen und zieht anschliessend das Lösungsmittel weitgehend ab. Das gebildete Zwischenprodukt kann isoliert werden, wird jedoch vorzugsweise direkt weiter verarbeitet.

In der nachfolgenden Reaktionsstufe wird das primär entstandene Umsetzungsprodukt der Verseifung unterworfen. Bevorzugt erfolgt die Verseifung unter basischen Bedingungen und dabei zweckmässigerweise unter basisch/alkoholischen Bedingungen. Geeignet ist zur Verseifung beispielsweise alkoholische Kali- oder Natronlauge. Die Verseifung wird vorzugsweise bei mässigen Temperaturen, z.B. im Bereich von 0°C vorgenommen. Die basische Komponente wird vorzugsweise im mehrfachen Überschuss über die eingesetzten Säuremengen verwendet. So kann beispielsweise die bei der Verseifung eingesetzte Basenmenge das Drei- bis Achtfache des stöchiometrischen Betrages, bezogen auf eingesetzte Steroidcarbonsäure, betragen. Mengen des Vier- bis Sechsfachen an Base sind hier bevorzugt.

Das Verseifungsprodukt wird nach Abschluss der Reaktion eingeengt und dann mit einem inerten, mit Wasser nicht mischbaren Lösungsmittel, beispielsweise Methylenchlorid, aufgenommen, gewaschen und dann zur Trockne eingeengt.

Als Reaktionsprodukt fällt auch hier die 20-Carbonylverbindung vom Progesterontyp unmittelbar an. Die intermediär wohl gebildete 17(20)-ungesättigte 20-Hydroxylverbindung lagert sich zur 20-Ketoverbindung um.

Die Ausgangsmaterialien der Erfindung fallen bei ihrer Herstellung häufig zusammen mit mehr oder weniger grossen Mengen an entsprechenden, jedoch 17(20)-gesättigten BNC-Verbindungen an. Es ist dabei im Rahmen der Erfindung möglich, die 17(20)-ungesättigten BNC-Verbindungen vor ihrer Umsetzung von den entsprechenden gesättigten Komponenten abzutrennen, man kann aber auch die Gemische der 17(20)-ungesättigten und hier gesättigten BNC-Verbindungen einer der beschriebenen Abbaureaktionen unterwerfen und die Auftrennung unter Gewinnung der 20-Carbonylverbindungen erst am Reaktionsprodukt vornehmen.

*Beispiel 1:*

*Curtius-Abbau eines Gemisches aus Δ 1,4,17-(20)-BNC (55%) und Δ 1,4-BNC (45%)*

1,7 g des genannten Säuregemisches in 10 ml trockenem $CH_2Cl_2$ werden bei 0°C mit 0,4 ml frisch über Squalen destilliertem Thionylchlorid versetzt und 20 min bei dieser Temperatur gerührt. Anschliessend engt man im Vakuum bei 0°C zur Trockne ein, nimmt den Rückstand wieder in $CH_2Cl_2$ auf und engt erneut zur Trockne ein.

Man löst das Gemisch der Säurechloride in 10-12 ml trockener $CH_2Cl_2$. Zu dieser Lösung gibt man bei 0°C 0,39 g $NaN_3$ in ca. 1,5 ml Wasser und 10 mg Tetrabutylammoniumchlorid als Phasentransferkatalysator. Es wird 20 min bei 0°C gerührt, danach versetzt man mit 2 ml Eiswasser, trennt die Phasen und wäscht mit wenig Eiswasser nach.

Die Lösung der Azide wird zu etwa 20 ml 70%iger Essigsäure getropft. Die Mischung wird vorsichtig auf 60-70°C erwärmt, wobei $CH_2Cl_2$ abdestilliert, und 30 min bei dieser Temperatur gehalten. Man versetzt danach mit 20 ml Wasser und engt im Vakuum zur Trockne ein.

Der Rückstand wird in 20 ml Methylenchlorid aufgenommen, man versetzt diese Lösung mit 20 ml 10%iger Natronlauge und rührt über Nacht. Nach der Phasentrennung wird die organische Phase neutral gewaschen, getrocknet und eingeengt. Es verbleiben 1,4 g kristalliner Rückstand, der im wesentlichen aus Pregna-1,4-dien-3,20-dion und 20-Amino-pregna-1,4-dien-3-on besteht.

Durch Digerieren dieses Rückstandes mit Ether lässt sich Pregna-1,4-dien-3,20-dion in der Etherphase anreichern. Filtration über eine kurze Kieselgelsäule (Laufmittel Essigester/Methylenchlorid 80-20) liefert 0,63 g (73% bezogen auf Δ 1,4,17-(20)-BNC) aminfreies, praktisch reines Pregna-1,4-dien-3,20-dion.

Die Gesamtausbeute an 20-Amino-pregna-1,4-dien-3-on, bezogen auf Δ 1,4-BNC, betrug 80% (durch quantitative DC aus dem Rohprodukt nach Überführung ins Acetamid bestimmt).

*Beispiel 2:*

*Carboxy-Inversions-Abbau eines Gemisches aus Δ 1,4,17(20)-BNC (55%) und Δ 1,4-BNC (45%)*

1,7 g des gesamten Carbonsäuregemisches werden nach Beispiel 1 zum Gemisch der Carbonsäurechloride umgewandelt. Die Lösung der Säurechloride in 10 ml absolutem Methylenchlorid wird auf −30°C gekühlt, dazu tropft man 1,0 g trockener m-Chlorperbenzoesäure (90%ig, Rest m-Chlorbenzoesäure) in 10 ml absolutem $CH_2Cl_2$ und schliesslich 0,48 g trockenes Pyridin in wenig abs. $CH_2Cl_2$ und lässt die Mischung über Nacht auf Raumtemperatur kommen.

Das Lösungsmittel wird im Vakuum weitgehend entfernt, den Rückstand versetzt man bei 0°C mit 12,5 ml 2N methanolischer Kalilauge und rührt danach mehrere Stunden bei Raumtemperatur. Anschliessend zieht man den grössten Teil des Methanols im Vakuum ab, nimmt den Rückstand in Methylenchlorid auf und wäscht die Methylenchloridphase nacheinander mit Wasser, verdünnter Schwefelsäure und wieder mit Wasser, trocknet und zieht das Lösungsmittel ab. Es verbleiben 1,4 g fast kristallinen Rückstands, der auf einer Kieselgelsäule chromatographiert wird. Mit Methylenchlorid/Essigester (3-30% Essigester) eluiert man nacheinander 0,13 g Pregna-1,4,20-trien-3-on (aus Δ 1,4-BNC), 0,53 g Pregna-1,4-dien-3,20-dion (aus Δ 1,4,17(20)-BNC) und 0,34 g 20-Hydroxy-pregna-1,4-dien-3-on.

**Patentansprüche**

1. Verfahren zur Herstellung von C20-Carbonyl-Steroiden durch Abbau von Steroid-C22-carbonsäuren, dadurch gekennzeichnet, dass man Δ 17(20)-Steroid-22-carbonsäuren als Ausgangsmaterial einsetzt und diese über ihr Säurehalogenid durch den Abbau nach Curtius oder den Carboxy-Inversions-Abbau unmittelbar zur 20-Carbonylverbindung umwandelt.

2. Verfahren nach Anspruch 1, dadurch gekennzeichnet, dass man Δ 4(5)- und gegebenenfalls weitere en-Bindungen in 1(2)- und/oder 9(11)-Stellung aufweisende BNC-Verbindungen mit der zusätzlichen en-Bindung in 17(20)-Stellung dem Verfahren unterwirft.

3. Verfahren nach den Ansprüchen 1 und 2, dadurch gekennzeichnet, dass man eine Sauerstoff-Funktion, insbesondere eine 11-Hydroxyl- oder

eine 11-Ketogruppe aufweisende BNC-Verbindungen der genannten Art einsetzt.

4. Verfahren nach den Ansprüchen 1 bis 3, dadurch gekennzeichnet, dass man die Steroidcarbonsäure über ihr Säurehalogenid in das Azid überführt, dabei die Umsetzung des Säurehalogenids zum Azid als wässerig/organische Zweiphasenreaktion unter Mitverwendung von quartären Ammoniumsalzen als Phasentransferkatalysatoren bei Temperaturen unterhalb etwa 25°C durchführt und das Azid unter Abspaltung von Stickstoff zur 20-Carbonylverbindung hydrolysiert.

5. Verfahren nach den Ansprüchen 1 bis 3, dadurch gekennzeichnet, dass man die Steroidcarbonsäure über ihr Säurehalogenid der Umsetzung mit Persäuren unterwirft und das Reaktionsprodukt zur 20-Carbonylverbindung verseift.

6. Verfahren nach Anspruch 5, dadurch gekennzeichnet, dass man mit m-Chlorperbenzoesäure arbeitet.

7. Verfahren nach den Ansprüchen 5 und 6, dadurch gekennzeichnet, dass man die Umsetzung des Steroidcarbonsäurehalogenids mit der Persäure bei −50°C bis Raumtemperatur zweckmässig in einem inerten Lösungsmittel und vorzugsweise in Gegenwart einer basischen Verbindung, insbesondere in Gegenwart von tertiären Aminen, durchführt.

## Claims

1. A process for the production of C20-carbonyl steroids by the degradation of steroid-C22-carboxylic acids, characterized in that Δ 17(20)-steroid-22-carboxylic acids are used as starting material and are directly transformed via their acid halide into the 20-carbonyl compound by Curtius degradation or by carboxy-inversion degradation.

2. A process as claimed in claim 1, characterized in that BNC-compounds containing Δ 4(5)- and optionally other ene-bonds in the 1(2)- and/or 9(11)-position, with the additional ene-bond in the 17(20)-position, are subjected to the process.

3. A process as claimed in claims 1 and 2, characterized in that BNC-compounds of the above-mentioned type containing an oxygen function, more especially an 11-hydroxyl or an 11-keto group, are used.

4. A process as claimed in claims 1 to 3, characterized in that the steroid carboxylic acid is converted via its acid alide into the azide, the reaction of the acid halide to form the azide being carried out as an aqueous/organic two-phase reaction using quaternary ammonium salts as phase transfer catalysts at temperatures below about 25°C, and the azide is hydrolyzed with elimination of nitrogen to form the 20-carbonyl compound.

5. A process as claimed in claims 1 to 3, characterized in that the steroid carboxylic acid is subjected via its acid halide to the reaction with peracids and the reaction product is hydrolyzed to form the 20-carbonyl compound.

6. A process as claimed in claim 5, characterized in that m-chloroperbenzoic acid is used.

7. A process as claimed in claims 5 and 6, characterized in that the reaction of the steroid carboxylic acid halide with the peracid is carried out at −50°C to room temperature, best in an inert solvent and preferably in the presence of a basic compound, more particularly in the presence of tertiary amines.

## Revendications

1. Procédé pour préparer des 20-carbonylstéroïdes par dégradation d'acides stéroïde-C22-carboxyliques, caractérisé en ce que l'on utilise en tant que produits de départ des acides Δ 17(20)-stéroïde-22-carboxyliques que l'on convertit directement en composés 20-carbonylés à partir de leurs halogénures d'acides par la dégradation selon Curtius ou par la dégradation avec inversion du groupe carboxyle.

2. Procédé selon la revendication 1, caractérisé en ce que l'on soumet au procédé des composés en BNC portant une liaison éthylénique en Δ 4(5) et éventuellement d'autres liaisons éthyléniques en 1(2) et/ou en 9(11) avec une liaison éthylénique supplémentaire en position 17(20).

3. Procédé selon les revendications 1 et 2, caractérisé en ce que l'on utilise des composés en BNC du type mentionné portant une fonction oxygénée, en particulier une fonction 11-hydroxy ou 11-céto.

4. Procédé selon les revendications 1 à 3, caractérisé en ce que l'on convertit l'acide stéroïde-carboxylique, par l'intermédiaire de son halogénure d'acide, en l'azide, en effectuant la conversion de l'halogénure d'acide en azide sous forme d'une réaction à deux phases aqueuse/organique avec utilisation conjointe de sels d'ammonium quaternaires en tant que catalyseurs de transfert de phase à des températures inférieures à 25°C environ, après quoi on hydrolyse l'azide avec séparation d'azote en le composé 20-carbonylé.

5. Procédé selon les revendications 1 à 3, caractérisé en ce que l'on soumet l'acide stéroïde-carboxylique, en passant par l'intermédiaire de son halogénure d'acide, à la réaction avec des peracides et on saponifie le produit de réaction en le composé 20-carbonylé.

6. Procédé selon la revendication 5, caractérisé en ce que l'on opère avec de l'acide m-chloroperbenzoïque.

7. Procédé selon les revendications 5 et 6, caractérisé en ce que l'on effectue la réaction de l'halogénure d'acide stéroïde-carboxylique avec le peracide à une température allant de −50°C jusqu'à la température ambiante, de préférence dans un solvant inerte et de préférence en présence d'un composé basique, en particulier en présence d'amines tertiaires.